# EUROPEAN PATENT SPECIFICATION

(11) **EP 0 710 105 B1**
(45) Date of publication and mention of the grant of the patent: **30.07.2003**
(21) Application number: 94918238.0
(22) Date of filing: 14.06.1994
(51) Int. Cl.: A61K 31/06, A61K 31/10, A61K 31/165, A61K 31/235, A61K 31/27, A61K 31/415, A61K 31/425, A61K 31/60, A61K 31/63, A61P 33/10

(54) **SYNERGISTIC ANTHELMINTIC COMPOSITIONS AGAINST FASCIOLA HEPATICA AND OTHER FASCIOLA SPECIES**
SYNERGISTISCHE ANTHELMINTISCHE ZUSAMMENSETZUNGEN GEGEN FASCIOLA HEPATICA UND ANDERE FASCOLA SPEZIEN
COMPOSITIONS ANTHELMINTIQUES SYNERGIQUES CONTRA FASCIOLA HEPATICA ET AUTRES ESPECES DE FASCIOLA

(30) Priority: 15.06.1993 AU PL933993
(43) Date of publication of application: 08.05.1996
(73) Proprietor: THE AUSTRALIAN NATIONAL UNIVERSITY, Acton, Australian Capital Territory 2601 (AU); The State of New South Wales, Camden, NSW 2570 (AU)
(72) Inventor: BORAY, Joseph Coloman, North Epping, NSW 2121 (AU)
(74) Representative: Woods, Geoffrey Corlett
(86) International application number: AU9400315
(87) International publication number: WO94028887

(56) References cited:
- EP-A- 0 101 412
- EP-A- 0 125 004
- WO-A-84/04455
- AU-A- 1 031 183
- US-A- 4 173 632
- DASH : "Control of helminthosis in lambs by strategic treatment with closantel and broad spectrum anthelmintics" AUSTR. VET. J. , vol. 63, no. 1, 1986, pages 4-7, XP002061762
- MCKELLAR ET AL.: "The pharmacology of flukicidal drugs" BR. VET. J., , vol. 147, no. 4, 1991, pages 306-321, XP002061763
- VETERINARY PARASITOLOGY, 26 (1988), pages 265-271, W.J. FOREYT: "Efficacy of a Fenbendazole - Triclabendazole combination against Fasciola hepatica and gastrointestinal nematodes in sheep".
- J. VET. PHARMACOL. THERAP. 8, (1985), pages 270-275, D.R. HENNESSY et al.: "Potentiation of the anthelmintic activity of oxfendazole by parbendazole".
- BR. VET. J. 125 (1969), pages 82-86, S.B. KENDALL et al.: "Synergism in the chemotherapy of Fascioliasis".
- MEDICINAL RESEARCH REVIEWS, Vol. 5, No. 3, (1985), pages 333-369, V.K. AGRAWAL et al.: "Combination Therapy in the treatment of helminth diseases", See pages 342, 346, 354-356, 359, 361.
- CHEMICAL ABSTRACTS, Volume 98 (15), 1983, No. 119237w, R. CAVIER et al.: "Pharmacological study on various antihelmenthic combinations"; & REV. MED. VET. (Toulouse), 1982, 133 (12), 779-783.
- CHEMICAL ABSTRACTS, Volume 80 (11), 1974, No. 55818e, K S NURTAEVA: "Experimental study of combinations of piperazine and sodium salts of phenasal with bithionol, trichlorophen and kamala during hymenolepidosis"; & MED. PARAZITOL. PARAZIT. BOLEZ. 1973, 42(5), 560-565.
- CHEMICAL ABSTRACTS, Volume 80 (5), 1974, No. 22702j, R L CAILLIER et al.: "Efficacy test of combined antihelmintics 3,5-diiodo-3'-chloro-4'-(p-chlorophenoxy) salicylanilide (Rafoxanide) and 2-(4'-thiazolyl) benzimidazole (thiabendazole) for sheep; & REV. MED. VET, 1973, 124 (6), 789-800.
- CHEMICAL ABSTRACT, Volume 76 (19), 1972, No. 108222g, E CHROUSTOVA: "Chemoprophylactic value of bis (2-hydroxy-3,5-dichlorphenyl) sulfoxide in combination with hexachlorophene against Fasciola hepatica infection under laboratory conditions"; & ACTA VET. (Brno), 1971, 40 (2), 215-219.

## Description

This invention relates to anthelmintic compositions, and in particular it relates to compositions of known anthelmintic active drugs exhibiting synergistic activity. These compositions may be used, for example, for the control of susceptible and resistant strains of immature and adult *Fasciola hepatica* or *F. gigantica* and for the prevention or management of anthelmintic resistance. Since all known drugs active against *F. hepatica* are also active against *F. gigantica* (Boray, 1986) the main target species will be referred to as *Fasciola spp*.

Fasciolosis caused by the liver flukes of herbivorous animals, *Fasciola spp.* may cause annual economic losses of 2,000 million dollars world wide. The strategic application of anthelmintic-active drugs alone cost the cattle and sheep industry approximately 60 to 80 million dollars annually. However, some anthelmintic-active drugs are only effective against the adult parasites requiring more frequent applications and some which were formerly effective against immature *F.hepatica* have now lost their advantage in the chemoprophylaxis of fasciolosis due to the development of drug resistance.

In Australia, drug resistance in immature *F.hepatica* to several anthelmintic drugs has been demonstrated in the field and in the laboratory (Boray and De Bono, 1989, Boray, 1990). It has been shown that long and regular use of salicylanilide compounds, particularly rafoxanide and closantel for the treatment of fasciolosis in sheep has selected resistant strains of *F.hepatica* in endemic areas of New South Wales. These strains have been shown to retain their resistant status in cattle and through several passages in sheep. Of seventeen isolates from different geographical regions, ten (58.8 %) showed resistance to rafoxanide at recommended dose rates in *F.hepatica* and side resistance to closantel was evident. Resistance to drugs was particularly manifested by immature *F.hepatica*, but rarely by the adult fluke.

The resistance of *F. hepatica* to anthelmintic-active drugs has been shown to be genetically controlled but the selection mechanism is unknown. Through preferential selection for resistance in the immature stage of *F.hepatica*, drugs effective against early immature fluke could lose their advantage in chemoprophylaxis of fasciolosis. Wide usage of a single highly-effective anthelmintic-active drug is undesirable since resistance may develop against several chemically unrelated drugs and more frequent treatments may be required to control fasciolosis. Thus, a strategic programme with alternating drug use and specific farm management should be implemented for the prevention of resistance or when resistance has been established or is suspected.

A study by Dash (Aust. Vet J., 1986, 63(1):4-7) found that a treatment schedule using broad spectrum anthelmintic and closantel administered concurrently was effective against *H. contortus* and *Trichostrongylus spp.* McKellar and Kinabo review flukicidal drugs marketed for the treatment of *F.hepatica* infestations in the UK in the British Veterinary Journal 1991, 147(4):306-321. US 4,173,632 discusses anthelmintic compositions which are especially useful for their fasciolicidal activity.

In the work leading to the present invention, the possibility of using a combination of anthelmintic-active drugs of different chemical groups at lower than their recommended dose rates was tested to ascertain whether drug synergism would lead to the successful treatment of acute fasciolosis caused by immature flukes of the disease due to resistant strains of *Fasciola hepatica* or to effective strategic treatment programmes for the prevention of resistance.

True synergism occurs when a combined efficacy of two or more chemotherapeutic agents is greater than the sum of their individual activities. Combination drugs are widely used in the chemotherapy of bacterial and parasitic protozoan pathogens. The combinations are particularly useful when development of resistance reduces the efficacy of individual chemotherapeutic agents but they are still highly effective in combinations. Drug combinations are most frequently employed for the control of bacterial diseases, malaria and insect pests, although true synergism rarely occurs amongst these combinations.

It is an object of the present invention to provide new combinations of known anthelmintic-active drugs for the treatment of infections with immature *F.hepatica*, to combat drug resistance in helminths and to reduce the likelihood of these parasites developing resistance.

Using synergistic combinations, three major advantages may be achieved:
(i) development of a product which is highly effective against susceptible and resistant immature and adult *Fasciola spp*. at reduced dose rates and possibly at reduced cost of production;
(ii) development of a product which is effective against a variety of fluke strains resistant to one or both components or which prevents the development of resistance; and
(iii) in certain compositions in which one or both of the active components have additional effect against parasitic infections other than fasciolosis, development of a product which would be suitable for the treatment of resistant and susceptible strains of liver flukes (*Fasciola spp.* and *Dicrocoelium dendriticum* ) as well as gastrointestinal nematodes, lungworms, tapeworms and *Oestrus ovis* in sheep.

In the present studies, the synergistic action of a number of combinations of anthelmintic-active drugs has been tested to ascertain the efficacy of these combinations against susceptible and resistant strains of parasites, or in the prevention of development of resistance. These studies have established that combinations of drugs from different chemical groups, such as triclabendazole and clorsulon, triclabendazole and luxabendazole, luxabendazole and clorsulon, closantel in combination with any of the above drugs, or nitroxynil combined with some of the above compounds, achieved high efficacy against susceptible strains of immature and adult *F. hepatica* or against those resistant to some of the above drugs, using a small fraction of their respective recommended dose rates by a synergistic effect.

According to the present invention, there is provided a composition for the control of Fasciola hepatica and other *Fasciola spp*. and other helminths in an animal, particularly in domestic animals and more particularly in ruminants such as sheep, goats, cattle or buffaloes, which comprises at least two anthelmintic-active, drugs, optionally together with an acceptable carrier or diluent, each of said anthelmintic-active drugs being selected from a different class of anthelmintic-active drugs, wherein said anthelmintic-active drugs are selected from:
triclabendazole + clorsulon;
triclabendazole + luxabendazole;
clorsulon + luxabendazole;
closantel + luxabendazole;
closantel + triclabendazole;
closantel + clorsulon;
nitroxynil + closantel;
nitroxynil + clorsulon; and
triclabendazole + oxfendazole

The present invention also provides a combined preparation of at least two anthelmintic-active drugs, optionally together with an acceptable carrier or diluent, for simultaneous or sequential use in a method for the control of *Fasciola hepatica* and other *Fasciola spp*., the anthelmintic-active drugs being those set out above.

In another aspect, the present invention also provides the use of at least two anthelmintic-active drugs, in the manufacture of a medicament for the control of *Fasciola hepatica* and other *Fasciola spp.* in animal, particularly in domestic animals and more particularly in ruminants. The medicament comprises at least two anthelmintic-active drugs optionally together with an acceptable carrier or diluent, each of said anthelmintic-active drugs being selected from a different class of anthehnintic-active drags, wherein said anthelmintic-active drugs are selected from:
triclabendazole + clorsulon;
triclabendazole + luxabendazole;
clorsulon + luxabendazole;
closantel + luxabendazole;
closantel + triclabendazole;
closantel + clorsulon;
nitroxynil + closantel;
nitroxynil + clorsulon;
triclabendazole + oxfendazole; and
closantel + oxfendazole.

In carrying out the method of the invention, the anthelmintic-active drugs may be administered to the animal simultaneously or sequentially to exert a synergistic effect in the animal, and where the drugs are administered sequentially they are administered at such times and in such a manner that they exert a synergistic effect in the animal.

Preferably, the anthelmintic-active drugs are administered orally to the animal, however other routes of administration such as parenteral or dermal administration may also be used.

In accordance with this invention, each of the anthelmintic-active drugs is selected from a known class of anthelmintic-active drugs. The drugs within the various classes which are encompassed within the present invention are as follows:
A. Halogenated monophenols and bisphenols
   Nitroxynil
B. Salicylanilides
   Closantel
C. Benzene sulfonamides
   Clorsulon
D. Halogenated Benzimidazoles
   Triclabendazole
E. Benzimidazole carbamates
   Luxabendazole
   Oxfendazole

(Full details of these known anthelmintic-active drugs are disclosed, for example, in "*Chemotherapy of Parasitic Diseases*", Ed. William C.Campbell and Robert S.Rew, Plenum Press, New York and London, 1986.)

Since the anthelmintic-active drugs used in the compositions of the present invention are known drugs, suitable effective doses and dose rates are already known. Preferably, in accordance with the present invention, each of the anthelmintic-active drugs is used at the known or recommended dose rate, or more preferably at a dose rate reduced below the recommended dose rate, for example, at a rate which is one half, one third or even one fifth of the recommended dose rate.

Particularly preferred combinations of anthelmintic-active drugs which have been shown to have advantageous activity in accordance with this invention include triclabendazole and clorsulon, closantel and triclabendazole, closantel and clorsulon, closantel and luxabendazole, clorsulon and luxabendazole, triclabendazole and luxabendazole, nitroxynil and closantel, nitroxynil and clorsulon, closantel and oxfendazole.

The combinations of triclabendazole (halogenated benzimidazole) and clorsulon (sulfonamide) at one fifth of their recommended dose rates, or triclabendazole and luxabendazole (benzimidazole carbamate) at one fifth and one third of their respective recommended dose rates, are highly effective against triclabendazole resistant immature *F.hepatica* by a true synergistic action. Similarly, a strong synergistic effect has been demonstrated when a combination of closantel (salicylanilide) at the recommended dose rate or at one third of the recommended dose with either triclabendazole (halogenated benzimidazole), clorsulon (sulfonamide), luxabendazole (benzimidazole carbamate) or nitroxynil (halogenated monophenol) at a fraction of their recommended dose rates are used successfully against strains of immature *F.hepatica* susceptible or resistant to either closantel, triclabendazole or luxabendazole.

Some of the above compounds, such as closantel, nitroxynil, luxabendazole and most other benzimidazole drugs, possess anthelmintic activity not only against liver flukes but also against gastrointestinal nematodes, such as *Haemonchus contortus*, lungworms and tapeworms. Accordingly, some of the synergistic combinations of the present invention are effective as broad spectrum anthelmintics for the control of simultaneously present trematode, cestode and nematode infections in domestic animals, particularly in ruminants.

Triclabendazole is a widely used drug which is highly effective against early immature and adult *F.hepatica* (Boray, 1986). The dose rate recommended by the manufacturer is 10 mg/kg for sheep. Closantel, a drug for the control of both *Haemonchus contortus* and *Fasciola spp.* with a particularly good persistent action against *H. contortus*, is widely used in helminth control programmes in areas where haemonchosis is endemic in the recommended dose rate of 7.5 t0 10 mg/kg (Hall et al., 1981). Luxabendazole is used for the control of gastro-intestinal nematodes and it is also highly effective against liver fluke (Boray, 1990). Clorsulon is a drug which is specific against *Fasciola spp*. used for cattle at 7 mg/kg which is highly effective against fluke aged 8 weeks or older but the suggested (not registered) dose rate against immature flukes for sheep is 15 mg/kg (Boray, 1986). It is also used for cattle as a subcutaneous injection at 2 mg/kg only against adult fluke. Nitroxynil is used mainly for cattle as a subcutaneous injection at a dose rate of 10 mg/kg but it is only effective against fluke aged 8 weeks or older.

Due to the occurrence of drug resistance in *F.hepatica* to salicylanilide compounds, such as rafoxanide and closantel, the resistance to benzimidazole carbamates such as luxabendazole (Boray, 1990), and the emerging resistance to triclabendazole, preventive action is required to reduce the chances of the development of resistance or for effective treatment for the resistant strains in the field. Resistance to closantel in both *F.hepatica* and *H.contortus* in the field is well documented (Boray, 1990, Rolfe, 1990). Because of the above considerations, in the work leading to the present invention triclabendazole, clorsulon, closantel and nitroxynil at a reduced dose rate were combined with other products to achieve efficacy against resistant strains.

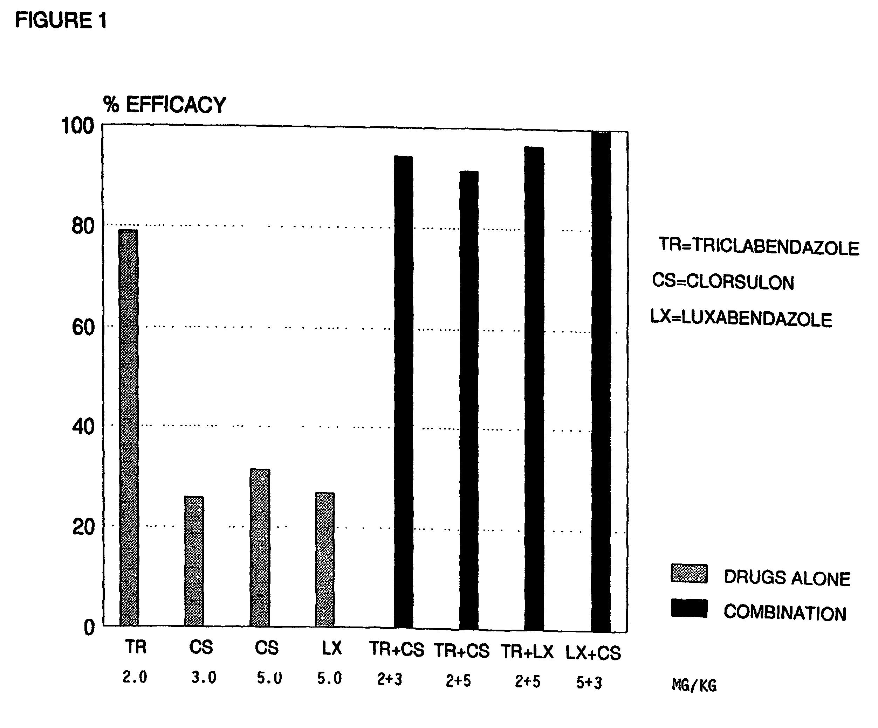

When the efficacy of the drugs belonging to different chemical groups was tested against a fully susceptible *F.hepatica* strain, one fifth of the recommended dose rate of triclabendazole combined with one third of the recommended dose rate of clorsulon or half of the recommended dose of luxabendazole showed high efficacy against immature fluke through a synergistic effect (Table 1 & Figure 1). At those dose rates efficacy would be expected only against adult flukes, since the efficacy of all known anthelmintics with the exception of diamphenetide is dose related and increased dose rates are necessary to kill early immature fluke (Boray, 1969). Such combinations developed for the treatment of susceptible strains of *Fasciola spp*. may reduce the cost of an anthelmintic preparation and would be effective against immature flukes. Because of the reduced dose rates, the tissue residues in sheep and cattle would be reduced. These combinations would also prevent the development of resistance in the field.

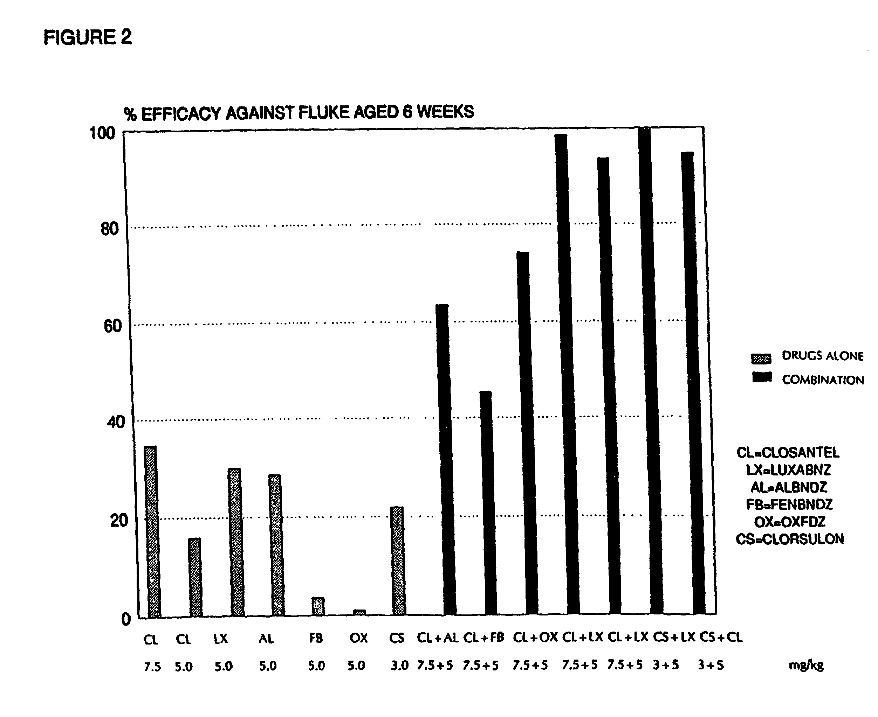

In the trials carried out with a *F. hepatica* strain resistant to salicylanilides such as closantel, but fully susceptible to all other drugs used, closantel at the Australian commercial dose rate of 7.5 mg/kg combined with oxfendazole at the recommended dose rate showed synergistic action but adjustment of the dose rate will be necessary to have higher efficacy against immature fluke. However when closantel at drastically reduced dose rate was combined with similarly reduced dose rates of luxabendazole or clorsulon, more than 94 % efficacy was achieved against immature *F. hepatica* (Table 2 & Figure 2). Those combinations with high efficacy would be effective against the most commonly occurring salicylanilide resistant fluke strains at reduced dose rates and would be also highly effective against most gastrointestinal nematodes (closantel+luxabendazole). The closantel+clorsulon combinations would be effective against salicylanilide resistant immature flukes and *H. contortus.* The triclabendazole+closantel combination (see Table 4) would be effective against multiple resistant immature flukes and *H. contortus*.

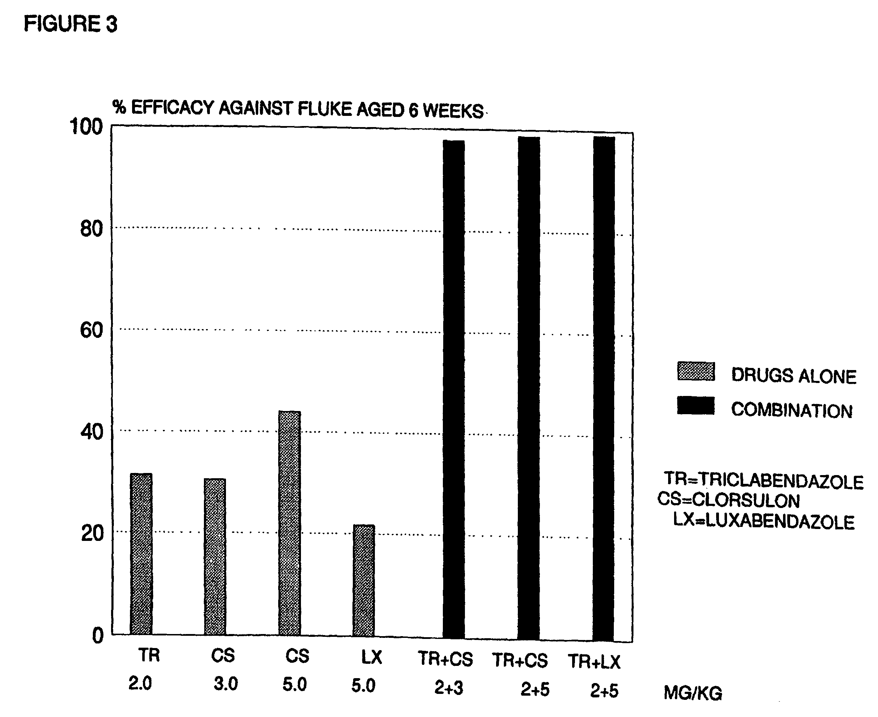

When efficacy was tested against a *F.hepatica* strain showing a degree of resistance against triclabendazole but susceptible to the other components, the combination of triclabendazole with clorsulon or luxabendazole gave high efficacy showing a true synergistic effect (Table 3 & Figure 3). Published data on the activity of luxabendazole against gastrointestinal nematodes is available (Kassai *et al*., 1988). Thus, this combination would be a suitable treatment for both resistant *F.hepatica* and gastrointestinal nematodes in sheep, whilst the combination of triclabendazole and clorsulon would give high efficacy against immature *Fasciola spp*. at drastically reduced dose rate.

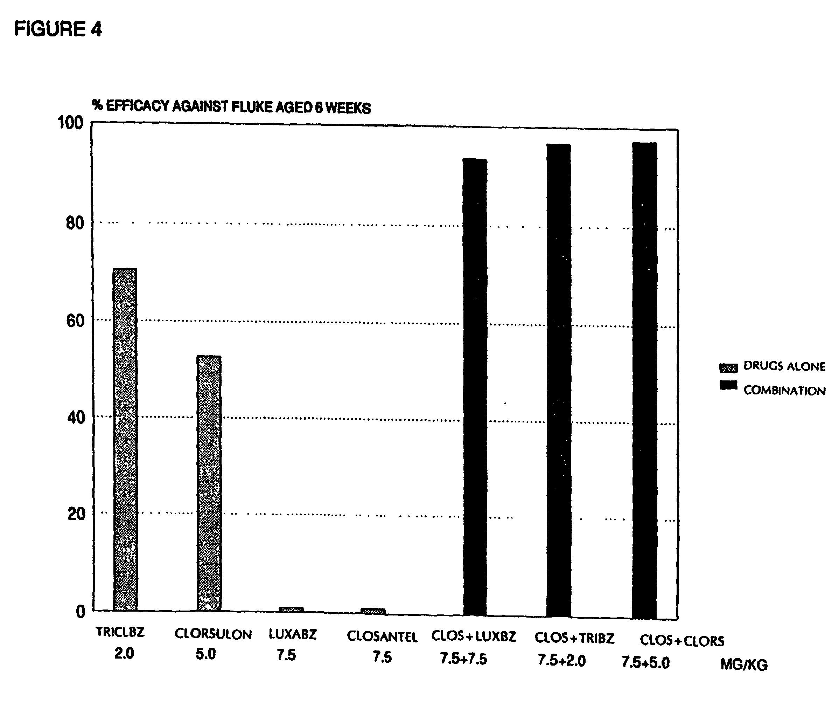

In the trials conducted with a *F.hepatica* strain resistant to both closante) and benzimidazole carbamates such as luxabendazole (Table 4 & Figure 4), high resistance of the strain was demonstrated when the recommended dose rate of either closantel or luxabendazole was ineffective. However, treatment with the combination of these two drugs belonging to different chemical groups was highly effective against immature fluke through a very strong synergistic effect. Combination of closantel with the chemically different triclabendazole or clorsulon also resulted in high efficacy, showing a strong synergistic effect against *F.hepatica*. Those combinations would be successful against some multiple resistant fluke strains and *H. contortus* at reduced dose rates.

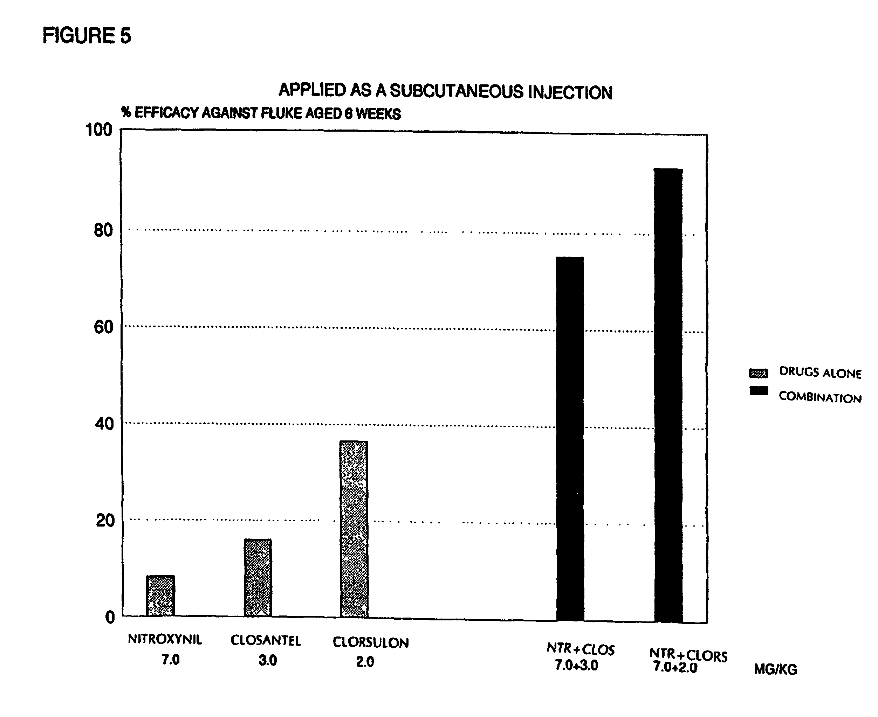

In a trial conducted with a strain of F. *hepatica* resistant to salicylanilides but susceptible to the other drugs used, the injectible nitroxynil, closantel and clorsulon alone gave low efficacy at reduced dose rates but high efficacy was achieved when nitroxynil was combined either with clorsulon or closantel showing strong synergism (Table 5 & Figure 5). Apart from the expected good activity against immature flukes at slightly adjusted dose rates, the nitroxynil+closantel combination would be useful since both are effective against *H. contortus* as well. The nitroxynil+clorsulon combination would provide very high efficacy against immature susceptible and salicylanilide resistant immature fluke and would also be effective against *H. contortus*.

Based on the above examples, synergistic combinations such as: triclabendazole + clorsulon; triclabendazole + luxabendazole; clorsulon + luxabendazole; closantel + luxabendazole; closantel + triclabendazole; closantel + oxfendazole; closantel + clorsulon; nitroxynil + closantel may be used to reduce the cost of anthelmintic drugs for the treatment or efficient chemoprophylaxis of fasciolosis, chiefly aimed at the elimination of early immature fluke, either by oral, parenteral or dermal application. The combination products may also be used to prevent the development of resistance and to control strains already resistant to any of the anthelmintic-active drugs used.

Some of the synergistic combinations such as: triclabendazole + luxabendazole, clorsulon + luxabendazole; triclabendazole + oxfendazole; closantel + oxfendazole; closantel + luxabendazole; closantel + clorsulon; nitroxynil + clorsulon; nitroxynil + closantel would synergise for higher efficacy against resistant and susceptible strains of immature *F.hepatica*. Some of the above combinations would also have a synergistic action against susceptible and resistant strains of *H. contortus*, since the mode of drug action and the mechanism of resistance in liver fluke and nematodes are similar. Synergistic effect would occur for efficacy against strains of *Haemonchus contortus* resistant to closantel or benzimidazoles. Some of them (luxabendazole, oxfendazole, closantel, nitroxynil in various combinations would also be effective against other gastrointestinal nematodes, tapeworms, *Dicrocoelium dendriticum* and *Oestrus ovis.*

Finally, where the composition includes luxabendazole or any other effective benzimidazole carbamate component, such as oxfendazole, the composition will also be effective against benzimidazole susceptible gastrointestinal nematodes, lungworms and tapeworms.

Further details of the present invention are set out, by way of illustration, in the following Example.

### EXAMPLE 1

### MATERIALS AND METHODS

### Sheep

In six controlled tests, a total of 212 merino wethers aged 1 to 2.5 years were used. The sheep were obtained from fluke free areas in North-West New South Wales.

### F.hepatica isolates.

Some isolates used in the trials were obtained from existing laboratory populations but the majority of the strains were isolated from the field, their resistance status was defined in several controlled tests and the isolates were then maintained in the laboratory. A laboratory strain from Compton U.K. (Compton Paddock Laboratories, Newbury, Berks. UK) was included in the tests. This strain has been maintained in the laboratory for many years and has not been exposed to anthelmintic treatment. The other isolates originated from properties where salicylanilide compounds were used with variable frequency for the control of fasciolosis or haemonchosis, and also various benzimidazole carbamates were used for the control of gastrointestinal nematodes. Those isolates were further selected in the laboratory with benzimidazole compounds, such as luxabendazole (Hoechst Pty.Ltd., Frankfurt am Main, Germany) or by 8 weekly dosing a group of sheep with triclabendazole (Fasinex, CIBA-GEIGY Aust.Ltd., Pendle Hill, NSW Australia) in a 2 acre paddock with natural snail habitats contaminated with *Fasciola hepatica .* The results of these trials were reported previously (Boray, 1990). Apart from the fully susceptible Compton strain, the other strains were resistant to salicylanilides and benzimidazole carbamates (Armidale "Morven" strain) or resistant to salicylanilides and a degree of resistance was detected against triclabendazole (P-16 or Paddock 16 strain), or only salicylanilides (Hampton II strain).

### Laboratory Procedures

In the trials, *F.hepatica* populations were isolated from properties and passaged through suitable intermediate host snails, *Lymnaea tomentosa, L.viridis* and *L.columella* in the laboratory. Sheep were then inoculated with the metacercariae of the selected strains by intraruminal injection of metacercariae suspended in 0.4% carboxy-methylcellulose solution. The sheep were divided into groups of 4 or 5. Standardised control tests (Boray, 1969), were carried out to determine the efficacy of various drugs and combinations against immature *F.hepatica* isolates.

### Statistical Tests for Synergism of Drugs:

Percentage reductions of fluke burdens for the sum of the individual drugs and for the combinations were estimated and their 95 % confidence limits were determined. Synergism was inferred, as indicated on the tables, when
a./ the lower 95 % limit for the combined formulation exceeded the percentage reduction estimate for the sum and
b./ the upper 95 % limit for the sum did not exceed the percentage reduction estimate for the combined formulation.

### Anthelmintic-active drugs.

The drugs tested were triclabendazole (Fasinex, CIBA-GEIGY Australia Ltd.), clorsulon (Curatrem, MSD AGVET Rahway, New Jersey, USA), luxabendazole (Hoechst Pty.Ltd., Frankfurt am Main, Germany), closantel (Seponver, SmithKline Animal Health Australia Ltd.), closantel injectible (Flukiver, Janssen Pharma, Belgium), clorsulon injectible (Ivomec F, MSD Agvet,Rahway, New Jersey, USA) and nitroxynil injectible (Trodax, Cyanamid-Webster Australia Ltd.) The commercial formulations were used and for uniform pharmacokinetic distribution they were injected intraruminally (for those drugs it is equal to oral dosing) at the selected dose rate according to the body weight of the sheep 6 weeks after inoculation. In the trials with the injectible nitroxynil the drugs were applied as a subcutaneous injection. For each trial a group of sheep were left untreated as controls. The sheep were slaughtered 16 weeks after inoculation and the flukes were recovered from the livers and counted. The number of flukes found at necropsy from the treated sheep were compared to those of the untreated controls (Boray, 1969). The dose rates were selected based on many preliminary studies on the efficacy against *F.hepatica* aged 6 weeks. In the present trials, substantially reduced subtherapeutic doses were used to demonstrate the synergistic effect of drugs. According to accepted standards (Boray, 1969) high efficacy is achieved when the drug reduces the fluke burden by 90% or more.

### RESULTS

The results of the trials are shown on Tables 1 to 5 and Figures 1 to 5 representing examples of synergistic activity of selected combinations of anthelmintic-active drugs.

Table 1 and Figure 1 show that when triclabendazole, clorsulon and luxabendazole alone at the low dose rates of 2, 3 to 5, and 5 mg/kg were used against a susceptible strain, their efficacy was 79, 25 to 31, and 26% respectively. When triclabendazole was given in combination with either the two low dose rates of clorsulon or that of luxabendazole, the high efficacy of 91 to 96% was achieved. When luxabendazole at 5 mg/kg was combined with clorsulon at 3 mg/kg the reduction of fluke burden was 99.7 %.

Table 2 and Figure 2 show that when closantel,luxabendazole, albendazole, oxfendazole, fenbendazole and clorsulon were given alone at low dose rates, very low efficacy was achieved. When closantel was combined with oxfendazole the efficacy was 74.2 % showing strong synergistic effect but this combination may require a slight increase of the dose rate to increase efficacy. When closantel was combined with luxabendazole or clorsulon at reduced dose rates the efficacy was more than 90 %. A combination of clorsulon and luxabendazole at 3 and 5 mg/kg respectively, almost 100 % efficacy occurred, which was achieved by strong synergistic effect.

Table 3 and Figure 3 show the results of experiments with the strain showing a degree of resistance against triclabendazole. The efficacy of triclabendazole at 2 mg/kg was only 30%. Clorsulon at 3 or 5 mg/kg and luxabendazole at 5 mg/mg gave efficacies of 30, 43 and 21 % respectively. When triclabendazole was combined with either clorsulon or luxabendazole, high efficacies of 98 to 99% were achieved.

Table 4 and Figure 4 show the results of experiment with the multiple resistant strain of Armidale-Morven showing a strong resistance in immature *F.hepatica* against closantel and luxabendazole. At the dose rate of 7.5 mg/kg, closantel and luxabendazole were inefficient, triclabendazole at 2 mg/kg and clorsulon at 5 mg/kg showed efficiency of 70 and 52 % respectively. When closantel was combined with luxabendazole, triclabendazole or clorsulon high efficacy of 93 to 97% was achieved through synergistic action.

Table 5 and Figure 5 show the results of the trial using a strain (Hampton II), resistant to salicylanilides but susceptible to all other drugs used. Nitroxynil at 7 mg/kg, closantel at 3 mg/kg and clorsulon at 2 mg/kg given as a subcutaneous injection reduced the number of flukes by only 8.3, 15.9 and 36.5 % respectively. When nitroxynil was combined with closantel or clorsulon at above dose rates, the efficacy was 75.2 and 93.1 % respectively, showing strong synergistic action. For practical application the dose rates of the nitroxynil-closantel combination should be slightly increased to achieve more than 90 % efficacy against immature fluke.

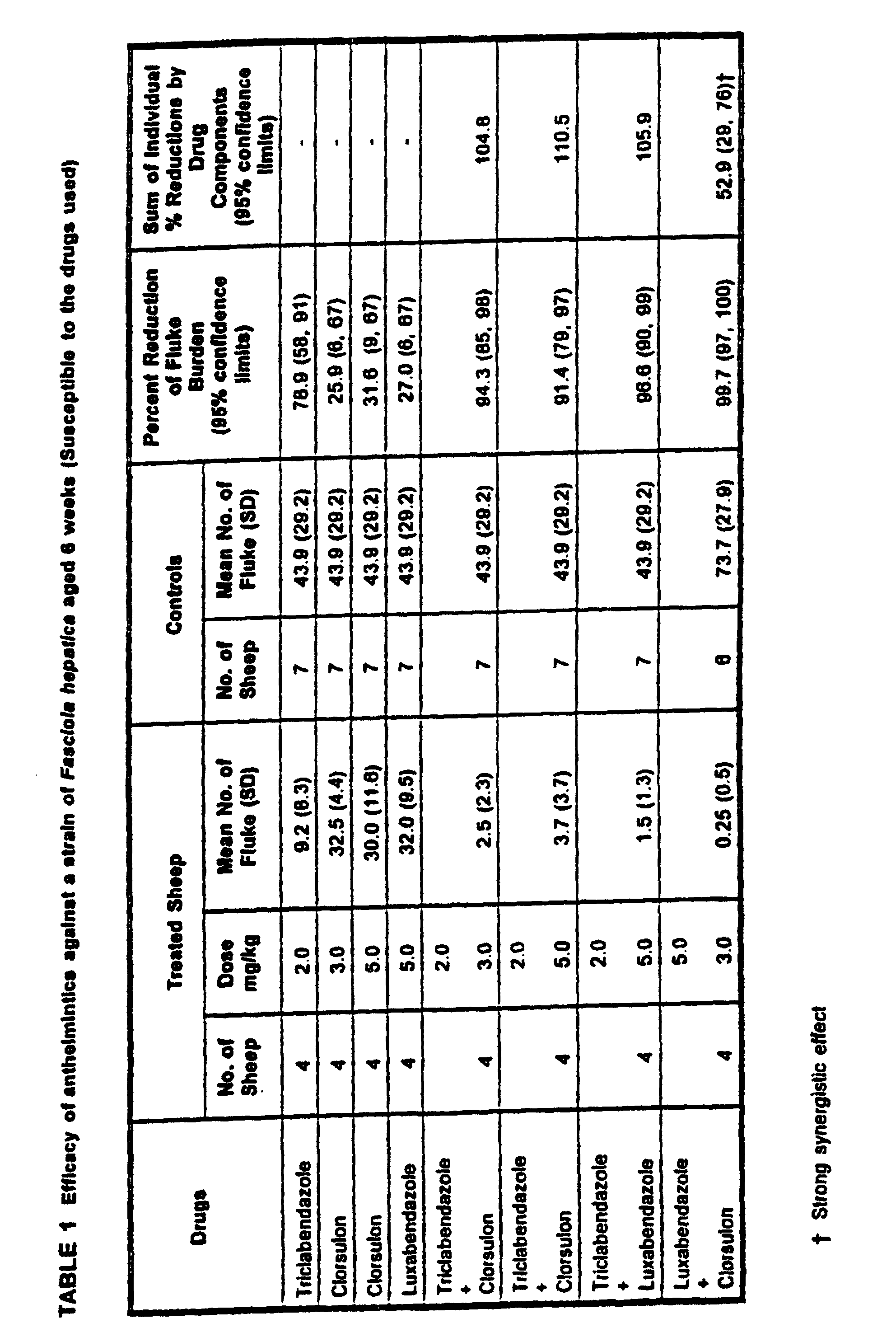

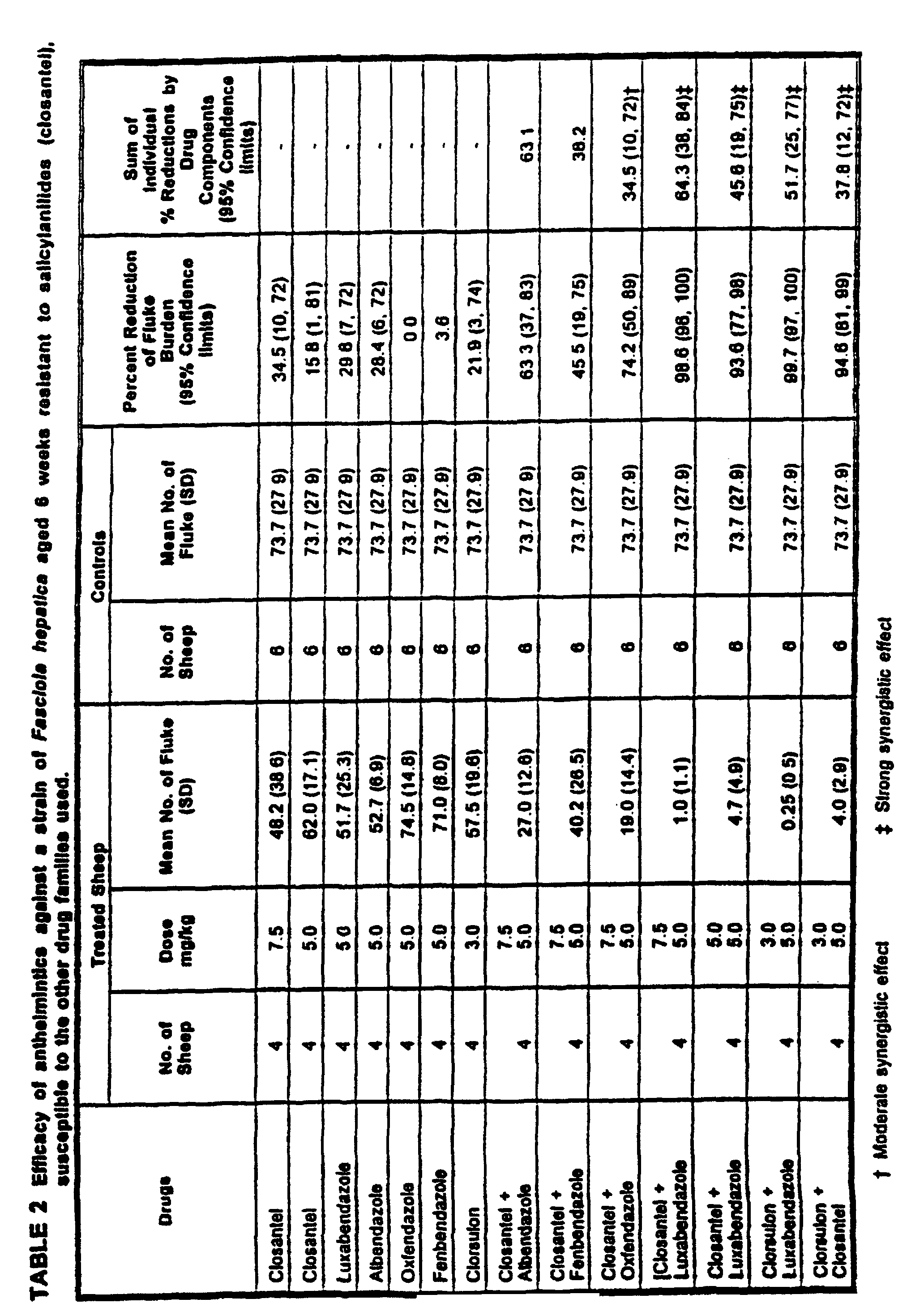

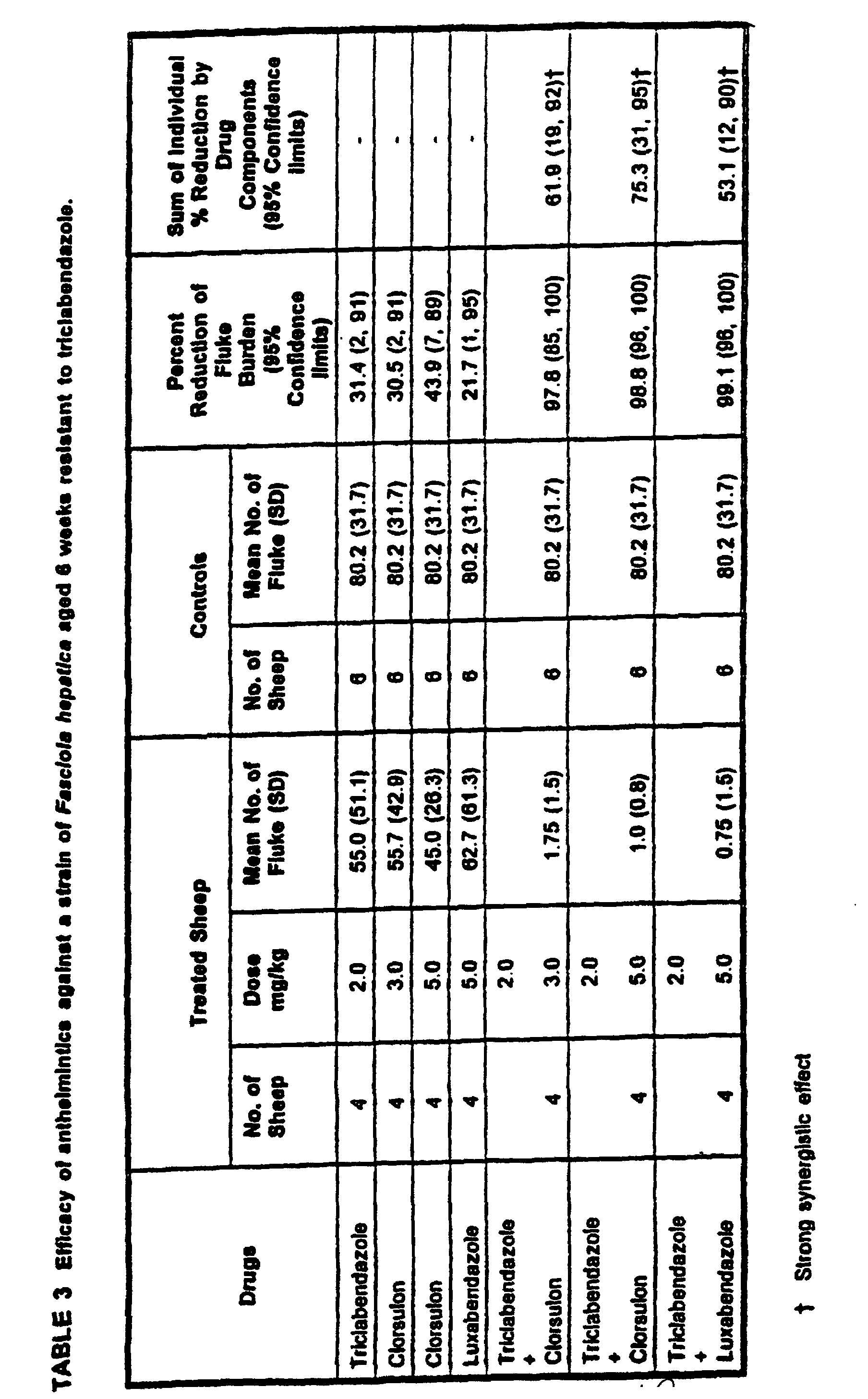

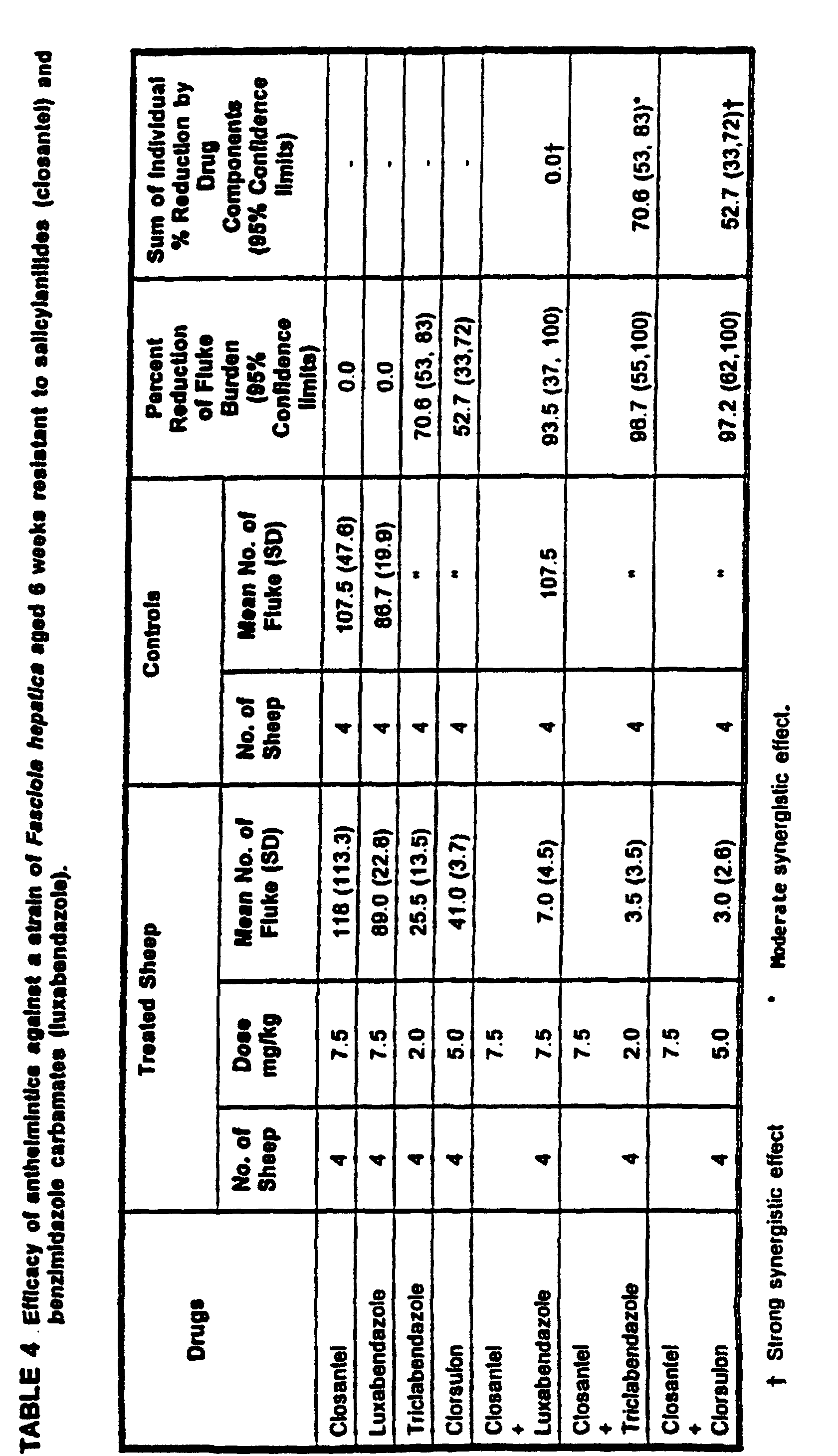

**TABLE 5**

| Efficacy of antheimintics administered as a subcutaneous injection against a strain of *Fasciola hepatica* aged 6 weeks, resistant to salicylanilides. | | | | | |
|---|---|---|---|---|---|
| Drugs | Treated Sheep | | | Percent Reduction of Fluke Burden (95% Confidence limits) | Sum of Individual % Reduction by Drug Components (95% Confidence Limits) |
| | No. of sheep | Dose mg/kg | Mean No. of Fluke (SD) | | |
| Nitroxynil | 5 | 7.0 | 26.6 (6.6) | 8.3 | - |
| Closantel | 5 | 3.0 | 24.4 (14.4) | 15.9 | - |
| Clorsulon | 5 | 2.0 | 18.4 (6.6) | 36.6 (9.76) | - |
| Nitroxynil + Closantel | | 7.0 | | | |
| | 5 | 3.0 | 7.2 (6.1) | 75.2 (49, 90) | 24.1 (3.79)* |
| Nitroxynil + Clorsulon | | 7.0 | | | |
| | 5 | 2.0 | 2.0 (2.0) | 93.1 (82, 97) | 44.8 (16, 77)† |
| Untreated controls | 4 | - | 29.0 (8.6) | - | - |

| | | | | | |
|---|---|---|---|---|---|
| † Strong synergestic effect | | | | | |
| * Moderate synergratic effect | | | | | |

### REFERENCES:

Boray, J.C. 1969. Experimental fascioliasis in Australia.
In *Advances in Parasitology.* (Edited by Dawes, B.) Vol.7, pp. 95-210, Academic Press, London and New York.

Boray, J.C. 1986. Trematode Infections of Domestic Animals.
In *Chemotherapy of Parasitic Diseases* (Edited by Campbell, W.C. and Rew, R.S.) pp.401-425. Plenum Publishing Corporation, New York, London, Tokyo.

Boray, J.C. and De Bono D. 1989. Drug resistance in *Fasciola hepatica.*
In *Advances in Veterinary Science* (Edited by Outteridge, P.M. and Richards, R.B. ) p.166. The Australian Veterinary Association, Artarmon, Australia.

Boray, J.C. 1990. Drug resistance in *Fasciola hepatica.*
In *Resistance of parasites to antiparasitic drugs.* (Edited by Boray, J.C., Martin P.J. and Ruosh, R.T. ) pp.51-60. MSD AGVET, Rahway, New Jersey.

Hall, C.A., Kelly, J.D., Whitlock, H.V. and Ritchie, L. 1981.
Prolonged anthelmintic effect of closantel and disophenol against a thiabendazole selected resistant strain of *Haemonchus contortus* in sheep. *Research in Veterinary Science ,* **31** : 104.

Kassai T., Takats, C., Fok, E. and Redl, P. 1988.
Activity of luxabendazole against liver flukes, gastrointestinal roundworms and lungworms in naturally infected sheep. *Parasitology Research* **75:** 14-18.

Rolfe, P.F. 1990. Resistance of *Haemonchus contortus* to broad and narrow spectrum anthelmintics.
In *Resistance of parasites to antiparasitic drugs.* (Edited by Boray, J.C., Martin, P.J. and Ruosh, R.T.) pp.115-122. MSD AGVET, Rahway, New Jersey.)

## Claims

1. A synergistic composition for the control of *Fasciola hepatica* and other *Fasciola spp*. in an animal, which comprises at least two anthelmintic-active drugs, optionally together with an acceptable carrier or diluent, each of said anthelmintic-active drugs being selected from a different class of anthelmintic-active drugs, wherein said anthelmintic-active drugs are selected from:
triclabendazole + clorsulon;
triclabendazole + luxabendazole;
clorsulon + luxabendazole;
closantel + luxabendazole;
closantel + triclabendazole;
closantel + clorsulon;
nitroxynil + closantel;
nitroxynil + clorsulon; and
triclabendazole + oxfendazole

2. A combined preparation of at least two anthelmintic-active drugs, optionally together with an acceptable carrier or diluent, for simultaneous or sequential use in a method for the control of *Fasciola hepatica* and other *Fasciola spp*. in an animal, each of said anthelmintic-active drugs being selected from a different class of anthelmintic-active drugs, wherein said anthelmintic-active drugs are selected from:
triclabendazole + clorsulon;
triclabendazole + luxabendazole;
clorsulon + luxabendazole;
closantel + luxabendazole;
closantel + triclabendazole;
closantel + clorsulon;
nitroxynil + closantel;
nitroxynil + clorsulon; and
triclabendazole + oxfendazole.

3. A preparation according to claim 2, wherein each of said anthelmintic-active drugs is administered at or below the recommended dose rate for that drug.

4. A preparation according to claim 2, wherein the animal is a domestic animal.

5. A preparation according to claim 2, wherein the animal is a sheep, goat, cow, buffalo or other ruminant animal.

6. A preparation according to claim 2, wherein said at least two anthelmintic-active drugs are administered to the animal simultaneously to exert a synergistic effect in the animal.

7. A preparation according to claim 2, wherein said at least two anthelmintic-active drugs are administered to the animal sequentially at such times and in such a manner to exert a synergistic effect in the animal.

8. A preparation according to any one of claims 2 to 7, wherein said anthelmintic-active drugs are administered orally, dermally or parenterally.

9. Use of at least two anthelmintic-active drugs optionally together with an acceptable carrier or diluent in the manufacture of a medicament for the control of *Fasciola hepatica* and other *Fasciola spp*. in an animal, each of said anthelmintic-active drugs being selected from a different class of anthelmintic-active drugs, wherein said anthelmintic-active drugs are selected from:
triclabendazole + clorsulon;
triclabendazole + luxabendazole;
clorsulon + luxabendazole;
closantel + luxabendazole;
closantel + triclabendazole;
closantel + clorsulon;
nitroxynil + closantel;
nitroxynil + clorsulon;
triclabendazole + oxfendazole; and
closantel + oxfendazole.

10. Use according to claim 9, wherein said medicament is for oral, dermal or parenteral administration.

## Patentansprüche

1. Synergistische Zusammensetzung zur Bekämpfung von *Fasciola hepatica* und anderen *Fasciola spp.* bei einem Tier, die mindestens zwei anthelminthisch wirksame Arzneimittel optional zusammen mit einem akzeptablen Träger oder Verdünnungsmittel umfasst, wobei jedes der anthelminthisch wirksamen Arzneimittel aus einer unterschiedlichen Klasse von anthelminthisch wirksamen Arzneimitteln ausgewählt ist, wobei die anthelminthisch wirksamen Arzneimittel ausgewählt sind aus:
Triclabendazol + Clorsulon;
Triclabendazol + Luxabendazol;
Clorsulon + Luxabendazol;
Closantel + Luxabendazol;
Closantel + Triclabendazol;
Closantel + Clorsulon;
Nitroxynil + Closantel;
Nitroxynil + Clorsulon; und
Triclabendazol + Oxfendazol.

2. Kombinationspräparat von mindestens zwei anthelminthisch wirksamen Arzneimitteln optional zusammen mit einem akzeptablen Träger oder Verdünnungsmittel zur gleichzeitigen oder aufeinanderfolgenden Verwendung bei einem Verfahren zur Bekämpfung von *Fasciola hepatica* und anderen *Fasciola spp*. bei einem Tier, wobei jedes der anthelminthisch wirksamen Arzneimittel aus einer unterschiedlichen Klasse von anthelminthisch wirksamen Arzneimitteln ausgewählt ist, wobei die anthelminthisch wirksamen Arzneimittel ausgewählt sind aus:
Triclabendazol + Clorsulon;
Triclabendazol + Luxabendazol;
Clorsulon + Luxabendazol;
Closantel + Luxabendazol;
Closantel + Triclabendazol;
Closantel + Clorsulon;
Nitroxynil + Closantel;
Nitroxynil + Clorsulon; und
Triclabendazol + Oxfendazol.

3. Präparat nach Anspruch 2, wobei jedes der anthelminthisch wirksamen Arzneimittel mit oder unter der empfohlenen Dosismenge für das Arzneimittel verabreicht wird.

4. Präparat nach Anspruch 2, wobei das Tier ein Haustier ist.

5. Präparat nach Anspruch 2, wobei das Tier ein Schaf, eine Ziege, eine Kuh, ein Büffel oder ein anderer Wiederkäuer ist.

6. Präparat nach Anspruch 2, wobei die mindestens zwei anthelminthisch wirksamen Arzneimittel dem Tier gleichzeitig zugeführt werden, wobei eine synergistische Wirkung auf das Lebewesen ausgeübt wird.

7. Präparat nach Anspruch 2, wobei die mindestens zwei anthelminthisch wirksamen Arzneimittel an das Tier nacheinander zu solchen Zeitpunkten und auf eine solche Weise verabreicht werden, dass eine synergistische Wirkung auf das Tier ausgeübt wird.

8. Präparat nach einem der Ansprüche 2 bis 7, wobei die anthelminthisch wirksamen Arzneimittel oral, dermal oder parenteral verabreicht werden.

9. Verwendung von mindestens zwei anthelminthisch wirksamen Arzneimitteln optional zusammen mit einem akzeptablen Träger oder Verdünnungsmittel zur Herstellung eines Medikaments zur Bekämpfung von *Fasciola hepatica* und anderen *Fasciola spp*. bei einem Tier, wobei jedes der anthelminthisch wirksamen Arzneimittel aus einer unterschiedlichen Klasse von anthelminthisch wirksamen Arzneimitteln ausgewählt ist, wobei die anthelminthisch wirksamen Arzneimittel ausgewählt sind aus:
Triclabendazol + Clorsulon;
Triclabendazol + Luxabendazol;
Clorsulon + Luxabendazol;
Closantel + Luxabendazol;
Closantel + Triclabendazol;
Closantel + Clorsulon;
Nitroxynil + Closantel;
Nitroxynil + Clorsulon;
Triclabendazol + Oxfendazol; und
Closantel + Oxfendazol.

10. Verwendung nach Anspruch 9, wobei das Medikament zur oralen, dermalen oder parenteralen Verabreichung dient.

## Revendications

1. Composition synergique pour la lutte contre *Fasciola hepatica* et d'autres espèces de *Fasciola* chez un animal, qui comprend au moins deux médicaments actifs antihelminthiques, éventuellement avec un support ou un diluant acceptable, chacun desdits médicaments actifs antihelminthiques étant choisi dans une classe différente de médicaments actifs antihelminthiques, lesdits médicaments actifs antihelminthiques étant choisis parmi :
triclabendazole + clorsulon ;
triclabendazole + luxabendazole ;
clorsulon + luxabendazole ;
closantel + luxabendazole ;
closantel + triclabendazole ;
closantel + clorsulon ;
nitroxynil + closantel ;
nitroxynil + clorsulon ; et
triclabendazole + oxfendazole.

2. Préparation combinée d'au moins deux médicaments actifs anti-helminthiques, éventuellement avec un support ou un diluant acceptable, pour une utilisation simultanée ou séquentielle dans une méthode de lutte contre *Fasciola hepatica* et d'autres espèces de *Fasciola* chez un animal, chacun desdits médicaments actifs antihelminthiques étant choisi dans une classe différente de médicaments actifs antihelminthiques, lesdits médicaments actifs antihelminthiques étant choisis parmi :
triclabendazole + clorsulon ;
triclabendazole + luxabendazole ;
clorsulon + luxabendazole ;
closantel + luxabendazole ;
closantel + triclabendazole ;
closantel + clorsulon ;
nitroxynil + closantel ;
nitroxynil + clorsulon ; et
triclabendazole + oxfendazole.

3. Préparation selon la revendication 2, dans laquelle chacun desdits médicaments actifs antihelminthiques est administré au taux de dose recommandé pour ce médicament ou en dessous de ce taux.

4. Préparation selon la revendication 2, dans laquelle l'animal est un animal domestique.

5. Préparation selon la revendication 2, dans laquelle l'animal est un mouton, une chèvre, une vache, un buffle ou un autre animal ruminant.

6. Préparation selon la revendication 2, dans laquelle lesdits au moins deux médicaments actifs antihelminthiques sont administrés simultanément à l'animal pour exercer un effet synergique chez l'animal.

7. Préparation selon la revendication 2, dans laquelle lesdits au moins deux médicaments actifs antihelminthiques sont administrés à l'animal successivement à des moments et de manière tels qu'ils exercent un effet synergique chez l'animal.

8. Préparation selon l'une quelconque des revendications 2 à 7, dans laquelle lesdits médicaments actifs antihelmintiques sont administrés par voie orale, dermique ou parentérale.

9. Utilisation d'au moins deux médicaments actifs antihelmintiques éventuellement avec un support ou un diluant acceptable dans la fabrication d'un médicament pour la lutte contre *Fasciola hepatica* et d'autres espèces de *Fasciola* chez un animal, chacun desdits médicaments actifs antihelminthiques étant choisi dans une classe différente de médicaments actifs antihelminthiques, lesdits médicaments actifs antihelminthiques étant choisis parmi :
triclabendazole + clorsulon ;
triclabendazole + luxabendazole ;
clorsulon + luxabendazole ;
closantel + luxabendazole ;
closantel + triclabendazole ;
closantel + clorsulon ;
nitroxynil + closantel ;
nitroxynil + clorsulon ;
triclabendazole + oxfendazole ; et
closantel + oxfendazole

10. Utilisation selon la revendication 9, dans laquelle ledit médicament est destiné à une administration orale, dermique ou parentérale.
